# EUROPÄISCHE PATENTSCHRIFT

(11) **EP 2 748 271 B1**
(45) Veröffentlichungstag und Bekanntmachung des Hinweises auf die Patenterteilung: **10.08.2016**
(21) Anmeldenummer: 11748945.0
(22) Anmeldetag: 25.08.2011
(51) Int. Cl.: C09K 3/14, A61K 8/02, A61K 8/25, A61K 8/31, A61K 8/92, A61K 9/00, A61K 47/02, A61K 47/44

(54) **STARRER FORMKÖRPER ZUR BEHANDLUNG VON JUCKREIZ**
RIGID MOULDED BODY FOR TREATING PRURITIS
CORPS MOULÉ ABRASIF CONTRE LES DÉMANGEAISONS

(43) Veröffentlichungstag der Anmeldung: 02.07.2014
(73) Patentinhaber: Beermann, Norbert, 80539 München (DE)
(72) Erfinder: Beermann, Norbert, 80539 München (DE)
(74) Vertreter: Ter Meer Steinmeister & Partner
(86) Internationale Anmeldenummer: PCT/EP2011/064635
(87) Internationale Veröffentlichungsnummer: WO 2013/026487

(56) Entgegenhaltungen:
- WO-A1-98/56520
- WO-A1-2006/101440
- DE-A1- 19 615 896
- US-B1- 6 235 070

## Beschreibung

### Gebiet der Erfindung

Die Erfindung betrifft einen starren Formkörper, welcher im Wesentlichen aus einer Vielzahl von aneinander haftenden, mit einer aus Wachs bestehenden Umhüllung versehenen Sandkörnern besteht, zur Beseitigung oder Linderung von Juckreiz. In einer besonderen Ausführungsform der Erfindung besteht das Wachs im Wesentlichen aus Bienenwachs.

### Stand der Technik

Juckreiz, medizinisch auch als Pruritus bezeichnet, ist eine unangenehme Empfindung der Haut, die Kratzen provoziert. Juckreiz kann grundsätzlich durch vielfältige Mediatoren ausgelöst werden, wie zum Beispiel durch Medikamente, Nahrungsmittel, Allergien, Pflanzen- oder Insektengifte oder Schädigung der Haut. In häufigen Fällen ist der Juckreiz das Symptom für eine Erkrankung. Chronische Formen des Pruritus sind oft therapieresistent.

Medikamente, die langfristig und sicher gegen Juckreiz helfen, sind nicht bekannt. Opioid-Antagonisten wie zum Beispiel Naltrexon^{®} konnten einige Erfolge erzielen. Bei lokalem Juckreiz hilft im allgemeinen Kühlung. Zum Teil hilft auch warmes Wasser, was jedoch zu trockener Haut und damit selbst wieder zu Juckreiz führen kann. Eine Lichttherapie mit UVB-Strahlen kann ebenfalls in manchen Fällen zu einer Linderung des Juckreizes führen. Die aus dem Stand der Technik bekannten Mittel und Methoden zur Behandlung von Juckreiz sind jedoch unbefriedigend, da sie häufig nur geringe oder nur temporäre Erfolge erzielen, der Juckreiz jedoch nach kurzer Zeit wieder auftritt oder aber, insbesondere bei der Behandlung mit Medikamenten, mit starken oder unerwünschten Nebenwirkungen verbunden sind.

Es besteht somit ein Bedarf nach verbesserten Mitteln zur Behandlung, d.h. Beseitigung oder Linderung, von Juckreiz, insbesondere nach Mitteln, welche zu einer langfristigen vollständigen Beseitigung oder Linderung von Juckreiz führen und frei von Nebenwirkungen sind.

Aus der DE 19 615 896 C2 ist ein starrer Sandkörper bekannt, bestehend aus einer Vielzahl von aneinander haftenden Sandkörnern, die mit einer aus Wachs bestehenden Umhüllung versehen sind. Durch Abreiben des Sandkörpers erhält man diskrete oder agglomerierte Sandkörner, die mit einer aus Wachs bestehenden Umhüllung versehen sind. Offenbart ist ferner die Verwendung des Sandkörpers als Sandreibe für die Hautmassage.

US 6 235 070 B1 sowie WO 98/56520 A1 betreffen einen starren Sandkörper, bestehend aus einer Vielzahl von aneinander haftenden und mit Wachs überzogenen Sandkörnern, sowie ein Verfahren zu dessen Herstellung, das dadurch gekennzeichnet ist, dass Sandkörner und Wachs getrennt voneinander bereitgestellt und auf eine Temperatur von 50-90 °C erwärmt, die erwärmten Sandkörner und das erwärmte, flüssige Wachs zusammengegeben werden, und die erhaltene Zusammensetzung zur Verfestigung abgekühlt wird. Die Verwendung des Sandkörpers zur Linderung oder Beseitigung von Juckreiz wird nicht beschrieben.

WO 2006/101440 A1 betrifft eine Materialzusammensetzung, die einerseits ein teilchenförmiges Material, und andererseits ein Bindemittel, das als Beschichtung auf den Materialpartikeln versehen ist, umfasst Die Verwendung der Materialzusammensetzung zur Linderung oder Beseitigung von Juckreiz wird nicht beschrieben.

### Aufgabe der Erfindung

Der Erfindung liegt die Aufgabe zugrunde, ein Mittel zur Verfügung zu Stellen, welches Juckreiz lindert oder beseitigt. Aufgabe der vorliegenden Erfindung ist ferner, ein Mittel zur Verfügung zu Stellen, welches Juckreiz lindert oder beseitigt und wobei der Juckreiz vermindernde oder beseitigende Effekt lange anhält und frei von unerwünschten Nebenwirkungen ist. Das Mittel sollte ferner einfach in der Handhabung, frei von gesundheitsgefährdenden Substanzen und somit auch für Kinder geeignet sein.

### Zusammenfassung der Erfindung

Durch die vorliegende Erfindung konnte überraschend festgestellt werden, dass der aus der DE 19 615 896 C2 bekannte Sandkörper (im Folgenden als Formkörper bzw. starrer Formkörper bezeichnet) geeignet ist, Juckreiz zu lindern oder zu beseitigen. Die der Erfindung zugrunde liegende Aufgabe wird somit gelöst durch einen starren Formkörper, bestehend aus einer Vielzahl von aneinander haftenden, mit einer aus Wachs bestehenden Umhüllung versehenen Sandkörnern, zur Verwendung bei der Behandlung von Juckreiz gemäß Anspruch 1. Vorteilhafte bzw. besonders zweckmäßige Ausführungsformen des Anmeldungsgegenstandes sind in den Unteransprüchen angegeben.

### Detaillierte Beschreibung der Erfindung

Gemäß der Erfindung hat sich überraschenderweise gezeigt, dass ein lediglich aus Sandkörnern und Wachs, insbesondere Bienenwachs, bestehendes Produkt, wobei das Wachs die Sandkörner vollständig oder teilweise umhüllt, in vielfältiger Weise zur Behandlung, also Beseitigung oder Linderung, von Juckreiz verwendet werden kann.

Das bei der Erfindung eingesetzte Wachs, welches die Vielzahl von Sandkörnern umhüllt, unterliegt keiner besonderen Beschränkung. Das Wachs kann somit ein natürliches, also tierisches oder pflanzliches Wachs, ein Mineralwachs, ein petrochemisches Wachs oder ein synthetisches oder chemisch modifiziertes Wachs sein. Das Wachs kann auch eine Mischung beliebiger Wachse sein, beispielsweise eine Mischung aus einem oder mehreren tierischen oder pflanzlichen Wachsen.

Beispiele für pflanzliche Wachse sind Candelilawachs, Carnaubawachs, Japanwachs, Espartograswachs, Korkwachs, Guarumawachs, Reiskeimölwachs, Zuckerrohrwachs, Ou-ricurywachs und Montanwachs. Beispiele für tierische Wachse sind Bienenwachs, Schellackwachs, Wollwachs, Walrat und Lanolin. Beispiele für Mineralwachse sind Ceresin und Ozokerrit beziehungsweise Erdwachs. Beispiele für petrochemische Wachse sind Petrolatum, Paraffnwachse, Stearinwachse und Mikrowachse. Beispiele chemisch modifizierter Wachse sind Hartwachse, wie Montanesterwachse, Sasolwachse und hydrierte Jojobawachse. Beispiele synthetischer Wachse sind Polyalkylenwachse und Polyethylenglykolwachse. Erfindungsgemäß bevorzugt ist Bienenwachs.

Das bei der Erfindung eingesetzte Wachs ist normalerweise bei Raumtemperatur knetbar, über 40°C ohne Zersetzung schmelzend, oberhalb des Schmelztemperaturbereichs verhältnismäßig niedrigviskos und nicht fadenziehend. In der Regel gehen Wachse etwa zwischen 50 und 90°C in den schmelzflüssigen, niedrigviskosen Zustand über. Bienenwachs wird im allgemeinen bei Temperaturen von 62 bis 65 °C flüssig.

Der in dem erfindungsgemäßen Formkörper enthaltende Sand unterliegt keiner besonderen Beschränkung. Die Sandkörner weisen üblicher Weise einen Durchmesser von 0,06 bis 3 mm auf und werden je nach Korngröße als Feinsand (Äquivalentdurchmesser von üblicherweise 0,06 bis 0,2 mm), Mittelsand (Äquivalentdurchmesser von üblicherweise 0,2 bis 0,63 mm) oder Grobsand (Äquivalentdurchmesser von üblicherweise 0,63 bis 3,0 mm) bezeichnet. Vorzugsweise wird Sand mit einem Durchmesser von 0,1 bis 0,5 mm eingesetzt. Beispielsweise kann Dünensand von Meeresstränden eingesetzt werden, dessen Körnchen üblicherweise einen Durchmesser von etwa 1 mm oder weniger aufweisen und der gegebenenfalls auf die gewünschte Korngröße zermahlen oder gesiebt werden kann.

Das Gewichtsverhältnis von Sand zu Wachs kann in geeigneter Weise etwa 1 : 1 bis 25: 1, bevorzugt 4: 1 bis 12: 1, besonders bevorzugt 6: 1 bis 10: 1, betragen.

Die in dem starren Formkörper enthaltenden Sandkörner sind vollständig oder teilweise mit der aus Wachs bestehenden Umhüllung versehen. Vorzugsweise sind die in dem erfindungsgemäßen Formkörper enthaltenden Sandkörner vollständig mit Wachs umhüllt.

Der erfindungsgemäße starre Formkörper besteht aus der Vielzahl von mit Wachs umhüllten Sandkörnern, wobei der erfindungsgemäße Formkörper jedoch auch wahlweise < 10 Gew.%, besonders bevorzugt < 5 Gew.%, an Zusatzstoffen enthalten kann, bezogen auf das Gesamtgewicht des erfindungsgemäßen Formkörpers. Erfindungsgemäß geeignete Zusatzstoffe sind beispielsweise kosmetische Zusätze wie hautpflegende Substanzen oder Antitranspirantien, Aromastoffe, Farbstoffe, Konsistenzgeber, ätherische und/ oder pflanzliche Öle, Vitamine und medizinische Wirkstoffe.

Die Herstellung des erfindungsgemäßen starren Formkörpers ist aus dem Stand der Technik bekannt und in der DE 19 615 896 C2 beschrieben. Am einfachsten erfolgt die Herstellung dadurch, dass man Sandkörner und Wachs getrennt voneinander auf eine Temperatur von 50-90°C erwärmt, die erwärmten Sandkörner und das erwärmte, flüssige Wachs zusammenfügt und die erhaltene Masse zur Verfestigung abkühlen lässt. Dieses Verfahren ermöglicht es, ohne Verwendung von Kunstharzen und irgendwelcher Lösungsmittel alleine durch Energiezuführung den Sand zu fixieren.

Der erfindungsgemäße starre Formkörper hat vorzugsweise Stiftform, besonders bevorzugt mit einem Durchmesser von 1 bis 3 cm und/oder einer Länge von 3 bis 10 cm. Bei einem dünneren Durchmesser als 1 cm neigt der starre Formkörper zum Zerbrechen, bei größerem Durchmesser als 3 cm wird der starre Formkörper unhandlich. In einer anderen Ausführungsform weißt der starre Formkörper Quaderform oder Kugelform auf, vorzugsweise mit einem Durchmesser von 5 bis 10 cm und/oder einer Länge von 5 bis 10 cm. Die gewünschte Form des erfindungsgemäßen Formkörpers kann beispielsweise dadurch erhalten werden, dass man bei dem vorgenannten Verfahren die erwärmten Sandkörner und das flüssige Wachs in entsprechenden Formen zusammenfügt oder die erhaltene Masse vor der Abkühlung in entsprechende Formen presst. Alternativ kann die gewünschte Form des Formkörpers aus der verfestigten Masse beispielsweise durch Ausstechen oder Schneiden erhalten werden.

Gemäß der vorliegenden Erfindung eignet sich der starre Formkörper zur Behandlung, d.h. Beseitigung oder Linderung, aller Arten von Juckreiz. Der Juckreiz kann hierbei als Begleiterscheinung von Hauterkrankungen hervorgerufen sein wie beispielsweise von Ekzemen oder Mykosen. Mögliche Auslöser von Juckreiz sind ferner Krankheiten wie Allergien, verursacht zum Beispiel durch Tierhaare, Blütenstaub, Milben, Chemikalien oder Nahrungsmittel, Neurodermitis, Diabetes oder Schuppenflechte. Juckreiz kann ferner durch altersbedingte degenerative Hautveränderungen ausgelöst sein, welche zu trockener oder rissiger Haut führen können. Andere mögliche Ursachen von Juckreiz sind beispielsweise Reizung der Haut wie sie u.a. durch mechanische Reizung wie Rasuren der Beine oder Arme hervorgerufen wird. Weitere Ursachen von Juckreiz sind Insektengifte wie beispielsweise nach Insektenbissen und Insektenstichen wie Mücken-, Bremsen-, Bienen- oder Wespenstichen.

Zur Beseitigung oder Linderung von Juckreiz wird der erfindungsgemäße starre Formkörper mit der juckenden Stelle in Kontakt gebracht, vorzugsweise wird er wiederholt unter leichtem Druck über die juckende Stelle gerieben. Insbesondere durch mehrmaliges Hin- und Herbewegen bzw. Reiben des starren Formkörpers über die juckende Hautstelle nimmt der Juckreiz immer weiter ab. Der starre Formkörper kann über einen Zeitraum von mehr als einer Minute, z.B. 1 bis 15 min, bevorzugt 2-10 min über die juckende Stelle gerieben werden. Für gewöhnlich ist der Juckreiz nach wenigen Minuten des Reibens ganz verschwunden. Vorzugsweise tritt der Juckreiz auch nach Beendigung er Behandlung, also nach Beendigung des Reibens mit dem starren Formkörper, nicht wieder auf. Normalerweise tritt der Juckreiz auch in einem Zeitraum von 24 Stunden nach Beendigung der Behandlung nicht wieder auf. Sollte der Juckreiz dennoch erneut wieder auftreten, kann die Behandlung wie oben beschrieben beliebig häufig wiederholt werden, ohne dass schädliche Nebenwirkungen auftreten.

Als positiver Nebeneffekt der oben beschriebenen Behandlung wird vielmehr ein angenehmer Massageeffekt erzielt. Insbesondere wenn der starre Formkörper unter verstärktem Druck über die juckende Stelle gerieben wird, kann ein die Durchblutung der behandelten Hautstelle fördernder Massageeffekt erzielt werden. Durch die verstärkte Durchblutung kann gegebenenfalls auch ein Heilungsprozess, z.B. wenn der Juckreiz durch eine entzündliche Erkrankung verursacht wird, gefördert werden.

Besonders vorteilhafte Effekte und Wirkungen werden erzielt, wenn die im erfindungsgemäßen Formkörper enthaltenden Sandkörner im Wesentlichen mit Bienenwachs umhüllt sind. "Im Wesentlichen" bedeutet, dass die Sandkörner vorzugsweise ausschließlich mit Bienenwachs umhüllt sind, jedoch auch geringe Menge anderer Wachse, wie beispielsweise die kostengünstigeren Stearin- oder Paraffinwachse, vorhanden sein können. Geringe Mengen sind hierbei vorzugsweise < 20 Gew.%, besonders bevorzugt < 5 Gew.%, bezogen auf das Gesamtgewicht des Wachses. Wird der Formkörper mit Bienenwachs hergestellt, ergibt sich für jedes einzelne Sandkorn eine teilweise oder vollkommene Umhüllung des Sandkorns mit Bienenwachs, im Allgemeinen im Mikrometerbereich. Der Vorteil von Bienenwachs gegenüber anderen Wachsen liegt darin, dass es sich bei Bienenwachs um ein Naturprodukt handelt. Bienenwachs ist somit ein die Umwelt und Ressourcen schonendes Produkt, welches im allgemeinen frei ist von chemischen und potentiell gesundheitsgefährdenden Zusatzstoffen.

Beim Vorgang des Reibens des starren Formkörpers der vorliegenden Erfindung wird der Formkörper mehr oder weniger stark auf die Haut gepresst. Durch das Hin- und Herbewegen des Formkörpers unter leichtem oder stärkerem Druck über die juckende Stelle löst sich das die Sandkörner umhüllende Bienenwachs ganz oder teilweise vom Sandkorn und kann sich somit in dünnen Schichten auf der Haut ablagern und teilweise in die Haut eindringen. Da natürliches Bienenwachs regelmäßig Spuren von heilenden Wirkstoffen enthält, wie beispielsweise Propolis, können diese Wirkstoffe bei Verwendung des Formkörpers in die Haut eindringen und so ihre positiven Wirkungen entfalten. Unter anderem wird Propolis antibiotische, antivirale und antimykotische Wirkung nachgesagt wird. Für diese positiven Wirkungen von Propolis werden insbesondere antioxidativ wirksame prenylierte Flavonoide verantwortlich gemacht. Bei Verwendung von Bienenwachs können somit in vorteilhafter Weise die vorhergehend genannten positiven Effekte erzielt werden.

Durch die Anwendung des starren Formkörpers wie oben beschrieben kann das Wachs, insbesondere das Bienenwachs, ohne große technische Hilfsmittel auf die Haut aufgetragen und hierbei äußerst sparsam dosiert werden. Durch die gleitende und schmierende Wirkung des Wachses geschieht dieses ohne starke Reibung, so dass die juckende Hautstelle geschont und nicht weiter gereizt wird. Auch bei Anwendung unter stärkerem Druck, wenn gleichzeitig ein Massageeffekt erzielt werden soll, wird die Haut nicht gereizt oder geschädigt. Insbesondere durch die hautschonende Anwendung, welche frei von schädlichen Nebenwirkungen ist, und die einfache Handhabung ist der erfindungsgemäße Formkörper nicht nur für Erwachsene, sondern auch für Kinder geeignet.

Ohne an eine Theorie gebunden zu sein, geschieht der Abrieb des Wachses vom Sandkorn auf die Haut aus dem Grund so leicht, weil beim Abrieb etwas Reibungshitze entsteht, und zwar sowohl auf der zu behandelnden Haut als auch beim Sandkorn. Aufgrund dieser gleichzeitigen Erwärmung des mit Wachs umgebenden Sandkorns und der durch die leichte Reibung erwärmten Haut bleibt das Wachs auf der Haut kleben und kann in die Haut eindringen. Die Sandkörner lösen sich hierbei zum Teil von dem Formkörper, so dass die Sandkörner nach und nach von dem Formkörper abperlen. Ein derartiger Effekt könnte bei einem reinen Wachsklumpen wie einem reinen Bienenwachsklumpen, d.h. ohne die gleichzeitige Anwesenheit der mit Wachs umhüllten Sandkörner, nicht erfolgen. Hierbei könnte der Abrieb nicht wirkungsvoll funktionieren, da nicht genug Reibung entsteht und somit weder die Haut noch der Wachsklumpen warm werden kann. Es könnte maximal ein Schmiereffekt auf der Haut entstehen.

Besonders gute Resultate bei der Behandlung von Juckreiz werden erzielt, wenn der starre Formkörper vor dem Inkontaktbringen mit der juckenden Stelle wenigstens partiell aufgeraut wird. Die juckende Stelle wird dann mit der aufgerauten Seite bzw. Fläche des Formkörpers wie oben beschrieben in Kontakt gebracht. Das Aufrauen geschieht vorzugsweise mit einem scharfen oder spitzen Gegenstand wie beispielsweise einem Messer. Durch das Aufrauen wird bereits bei leichtem Druck des Formkörpers auf der Haut ein Abrieb von Sandkörnern erzielt, so dass die Behandlung der juckenden Stelle mit dem entsprechend aufgerauten Formkörper für den Anwender besonders leicht, das heißt ohne große Kraftaufwendung, bewerkstelligt werden kann. Die Anwendung ist so besonders angenehm und wirksame Effekte können nach kürzerer Zeit, verglichen mit dem unaufgerauten Formkörper, erzielt werden.

Die Erfindung wird anhand der folgenden Ausführungsbeispiele näher erläutert.

### Beispiele

### Beispiel 1: Beseitigung von Juckreiz, hervorgerufen durch Pollenallergie

Eine Probandin, welche unter einer Pollenallergie leidet, spürte nach Kontakt mit verschiedenen Gräsern starken Juckreiz an den Stellen, welche mit den Gräsern in Kontakt gekommen waren. Nach Anwendung eines starren Formkörpers, welcher aus Sand und Bienenwachs in einem Massenverhältnis von 8:1 bestand, durch mehrminütiges Reiben auf den juckenden Stellen besserte sich der Juckreiz und verschwand nach ca. 10 bis 15 Minuten der Behandlung vollständig. Auch in den folgenden 24 Stunden trat der Juckreiz nicht wieder auf. Die Anwendung des Formkörpers gemäß der vorliegenden Erfindung führte somit zu einer vollständigen Beseitigung des Juckreizes.

### Beispiel 2: Beseitigung von Juckreiz, hervorgerufen durch Nahrungsmittelallergie

Ein unter einer Nahrungsmittelallergie leidender Proband spürte nach Einnahme verschiedener Fruchtsäfte einen starken Juckreiz an verschiedenen Stellen der Haut. Das Abreiben mit einem starren Formkörper, welcher aus Sand und Bienenwachs in einem Massenverhältnis von 8:1 bestand, über einen Zeitraum von 15 bis 20 Minuten bewirkte das vollständige Verschwinden des Juckreizes. Der Juckreiz trat auch in den folgenden 24 Stunden nicht wieder auf. Die Anwendung des starren Formkörpers der vorliegenden Erfindung führte somit zu einem vollständigen Verschwinden des Juckreizes.

### Beispiel 3: Beseitigung von Juckreiz hervorgerufen durch Insektenstich

Ein Proband spürte nach dem Erleiden von mehreren Insektenstichen an verschiedenen Stellen des Körpers starken Juckreiz an den Einstichstellen. Nach Reiben eines aus Sand und Bienenwachs in einem Massenverhältnis von 8:1 bestehenden starren Formkörpers besserte sich der Juckreiz und verschwand nach 15 Minuten der Anwendung vollständig. Der Juckreiz trat an den Einstichstellen auch in den nächsten 24 Stunden nicht wieder auf. Die Anwendung des Formkörpers der vorliegenden Erfindung führte somit zu einer vollständigen Beseitigung des Juckreizes.

### Beispiel 4: Beseitigung von Juckreiz, hervorgerufen durch mechanische Hautreizung (Rasur)

Eine Probandin spürte nach Rasur der Beine ein brennendes und juckendes Gefühl auf der Haut, welche durch die Einwirkung der Rasur gereizt war, wie durch Rötung der Haut zu erkennen war. Nach mehrminütigem Reiben eines aus Sand und Bienenwachs in einem Massenverhältnis von 8:1 bestehenden Formkörpers über die rasierten Stellen verschwand das subjektive Gefühl des Juckens und Brennens vollständig. Die Anwendung des starren Formkörpers hatte somit die gereizte Haut beruhigt und zu einem vollständigen Verschwinden des brennenden und juckenden Gefühls geführt.

### Beispiel 5: Beseitigung von Juckreiz, hervorgerufen durch Diabetes

Ein unter Diabetes leidender Proband verspürte nach Aufnahme kalorienhaltiger Nahrung starken Juckreiz an den Füßen. Nach mehrminütigem Reiben eines aus Sand und Bienenwachs in einem Massenverhältnis von 8:1 bestehenden Formkörpers über die juckenden Stellen ließ der Juckreiz nach und verschwand nach ca. 10 bis 15 Minuten vollständig. Auch innerhalb der folgenden 24 Stunden trat der Juckreiz nicht wieder auf. Die Verwendung des Formkörpers führte somit zu einem vollständigen Verschwinden des Juckreizes.

### Beispiel 6: Beseitigung von Juckreiz, hervorgerufen durch Insektenstich

Eine Probandin verspürte nach Erleiden mehrerer Insektenstiche starken Juckreiz an den Einstichstellen. Nach mehrminütigem Reiben eines aus Sand und Bienenwachs in einem Massenverhältnis von 8:1 bestehenden Formkörpers über die juckenden Stellen ließ der Juckreiz nach und verschwand nach ca. 10 bis 15 Minuten vollständig. Auch innerhalb der folgenden 24 Stunden trat der Juckreiz nicht wieder auf. Die Verwendung des Formkörpers führte somit zu einem vollständigen Verschwinden des Juckreizes.

### Beispiel 7: Beseitigung von Juckreiz, hervorgerufen durch Hautallergie

Ein unter Hautallergie leidender Proband verspürte starken Juckreiz an Schulter und Rücken. Nach mehrminütigem Reiben eines aus Sand und Bienenwachs in einem Massenverhältnis von 8:1 bestehenden Formkörpers über die juckenden Stellen ließ der Juckreiz nach und verschwand nach ca. 10 bis 15 Minuten vollständig. Auch innerhalb der folgenden 24 Stunden trat der Juckreiz nicht wieder auf. Die Verwendung des Formkörpers führte somit zu einem vollständigen Verschwinden des Juckreizes.

### Beispiel 8: Beseitigung von Juckreiz, hervorgerufen durch Insektenstich, bei Kindern

Zwei Kinder im Alter von 10 und 12 Jahren verspürten nach Erleiden von Insektenstichen starken Juckreiz an den Einstichstellen. Nach selbständigen mehrminütigem Reiben eines aus Sand und Bienenwachs in einem Massenverhältnis von 8:1 bestehenden Formkörpers über die juckenden Stellen ließ der Juckreiz nach und verschwand nach ca. 10 bis 15 Minuten vollständig. Auch innerhalb der folgenden 24 Stunden trat der Juckreiz nicht wieder auf. Die Verwendung des Formkörpers führte somit zu einem vollständigen Verschwinden des Juckreizes. Dieses Beispiel verdeutlicht ferner, dass der erfindungsgemäße Formkörper derart einfach in der Handhabung ist, dass er problemlos von Kindern angewendet werden kann.

Wie durch die vorhergehenden Beispiele gezeigt, führt die Anwendung des starren Formkörpers der vorliegenden Erfindung zur Linderung und vollständigem Verschwinden von Juckreiz hervorgerufen durch verschiedene Ursachen wie hierin aufgeführt. Die Verwendung des starren Formkörpers ist ferner einfach in der Handhabung, frei von Nebenwirkungen sowie gesundheitsgefährdenden Substanzen und schont die Haut. Hierdurch ist der Formkörper auch für die Anwendung bei Kindern geeignet.

## Patentansprüche

1. Starrer Formkörper, bestehend aus einer Vielzahl von aneinander haftenden, mit einer aus Wachs bestehenden Umhüllung versehenen Sandkörnern sowie wahlweise weniger als 10 Gew.%, bezogen auf das Gesamtgewicht des Formkörpers, an Zusatzstoffen gewählt aus kosmetischen Zusätzen einschließlich hautpflegenden Substanzen oder Antitranspirantien, Aromastoffen, Farbstoffen, Konsistenzgebern, ätherischen und/oder pflanzlichen Ölen, Vitaminen und medizinischen Wirkstoffen, zur Verwendung bei der Behandlung von Juckreiz.

2. Starrer Formkörper zur Verwendung nach Anspruch 1, wobei die Behandlung die Beseitigung oder Linderung von Juckreiz umfasst.

3. Starrer S Formkörper zur Verwendung nach Anspruch 1 oder 2, wobei das Wachs ausgewählt ist aus tierischem Wachs, pflanzlichem Wachs, Mineralwachs, petrochemischem Wachs, synthetischem oder chemisch modifiziertem Wachs oder Mischungen davon.

4. Starrer Formkörper zur Verwendung nach einem der Ansprüche 1 bis 3, wobei das Wachs ein tierisches Wachs ist, vorzugsweise ausgewählt aus Bienenwachs, Schellackwachs, Wollwachs, Walrat, Lanolin oder Mischungen davon, besonders bevorzugt Bienenwachs.

5. Starrer Formkörper zur Verwendung nach einem der Ansprüche 1 bis 3, wobei das Wachs ein pflanzliches Wachs ist, vorzugsweise ausgewählt aus Candelilawachs, Camaubawachs, Japanwachs, Espartograswachs, Korkwachs, Guarumawachs, Reiskeimöl wachs, Zuckerrohrwachs, Ou-ricurywachs, Montanwachs oder Mischungen davon.

6. Starrer Formkörper zur Verwendung nach einem der Ansprüche 1 bis 3, wobei das Wachs ein petrochemisches Wachs ist, vorzugsweise Paraffinwachs, Stearinwachs oder Mischungen davon.

7. Starrer Formkörper zur Verwendung nach einem der vorhergehenden Ansprüche, wobei die mit Wachs umhüllten Sandkörner ein Gewichtsverhältnis von Sand zu Wachs von 2: 1 bis 25: 1, bevorzugt 4: 1 bis 12: 1, besonders bevorzugt 6: 1 bis 10: 1, aufweisen.

8. Starrer Formkörper zur Verwendung nach einem der vorhergehenden Ansprüche, wobei die Sandkörner einen Durchmesser von 0,06 bis 3 mm, vorzugsweise von 0,1 bis 0,5 mm, aufweisen.

9. Starrer Formkörper zur Verwendung nach einem der vorhergehenden Ansprüche, wobei der Juckreiz ausgelöst ist durch Insektenstich, Allergie, Neurodermitis, Hautschädigung und/oder -reizung, Nesselkontakt und/oder Diabetes.

10. Starrer Formkörper zur Verwendung nach einem der vorhergehenden Ansprüche, wobei der Formkörper Stiftform aufweist, vorzugsweise mit einem Durchmesser von 1 bis 3 cm und/oder einer Länge von 3 bis 10 cm.

11. Starrer Formkörper zur Verwendung nach einem der vorhergehenden Ansprüche, wobei der starre Formkörper eine Quaderform oder Kugelform aufweist, vorzugsweise mit einem Durchmesser von 5 bis 10 cm und/oder einer Länge von 5 bis 10 cm.

12. Starrer Formkörper zur Verwendung nach einem der vorhergehenden Ansprüche, wobei der Formkörper vor dem Inkontaktbringen mit der juckenden Stelle wenigstens partiell aufgeraut wird.

## Claims

1. A rigid shaped article consisting of a plurality of mutually adhering grains of sand provided with a coating consisting of wax as well as optionally less than 10% by weight with respect to the total weight of the shaped article of additives selected from cosmetic additives including skin care substances or antiperspirants, fragrances, colorants, consistency enhancers, essential and/or vegetable oils, vitamins and medically active ingredients, for use in the treatment of itching.

2. The rigid shaped article for use as claimed in claim 1, wherein the treatment comprises the elimination or relief of itching.

3. The rigid shaped article for use as claimed in claim 1 or 2, wherein the wax is selected from animal wax, vegetable wax, mineral wax, petrochemical wax, synthetic or chemically modified wax or mixtures thereof.

4. The rigid shaped article for use as claimed in any of claims 1 to 3, wherein the wax is an animal wax, preferably selected from beeswax, shellac wax, wool wax, spermaceti, lanolin or mixtures thereof, particularly preferably beeswax.

5. The rigid shaped article for use as claimed in any of claims 1 to 3, wherein the wax is a vegetable wax, preferably selected from candelilla wax, carnauba wax, Japan wax, esparto grass wax, cork wax, guaruma wax, rice oil wax, sugar cane wax, ouricury wax, montan wax or mixtures thereof.

6. The rigid shaped article for use as claimed in any of claims 1 to 3, wherein the wax is a petrochemical wax, preferably paraffin wax, stearin wax or mixtures thereof.

7. The rigid shaped article for use as claimed in one of the preceding claims, wherein the grains of sand coated with wax exhibit a weight ratio of sand to wax of 2:1 to 25:1, preferably 4:1 to 12:1, particularly preferably 6:1 to 10:1.

8. The rigid shaped article for use as claimed in one of the preceding claims, wherein the grains of sand have a diameter of 0.06 to 3 mm, preferably 0.1 to 0.5 mm.

9. The rigid shaped article for use as claimed in one of the preceding claims, wherein the itching is triggered by insect bites, allergies, neurodermatitis, skin damage and/or irritation, contact with nettles and/or diabetes.

10. The rigid shaped article for use as claimed in one of the preceding claims, wherein the shaped article is in the form of a stick, preferably with a diameter of 1 to 3 cm and/or a length of 3 to 10 cm.

11. The rigid shaped article for use as claimed in one of the preceding claims, wherein the rigid shaped article has a cuboid or spherical shape, preferably with a diameter of 5 to 10 cm and/or a length of 5 to 10 cm.

12. The rigid shaped article for use as claimed in one of the preceding claims wherein, prior to bringing the shaped article into contact with the itching region, the shaped article is at least partially roughened.

## Revendications

1. Pièce moulée rigide, étant composé d'une multitude de grains de sable pourvus d'un enrobement de cire adhérant les uns aux autres ainsi que facultativement de moins que 10% en poids par rapport au poids total de la pièce moulée d'adjuvants choisis parmi des adjuvants cosmétiques incluant des substances soignant la peau ou des antitranspirants, des substances aromatiques, des colorants, des donneurs de consistence, des huiles essentielles et/ou végétales, des vitamines et des agents actifs médicinaux pour usage dans le traitement de la démangeaison.

2. Pièce moulée rigide pour usage selon la revendication 1, dans laquelle le traitement comprend l'élimination ou le soulagement de la démangeaison.

3. Pièce moulée rigide pour usage selon la revendication 1 ou 2, dans laquelle la cire est choisie parmi une cire animale, une cire végétale, une cire minérale, une cire pétrochimique, une cire synthétique ou chimiquement modifiée, ou les mélanges de ceux-ci.

4. Pièce moulée rigide pour usage selon l'une quelconque des revendications 1 à 3, dans laquelle la cire est une cire animale, de préférence choisie parmi la cire d'abeille, la cire de gomme-laque, la cire de laine, le blanc de baleine, la lanoline ou les mélanges de ceux-ci, particulièrement préféré la cire d'abeille.

5. Pièce moulée rigide pour usage selon l'une quelconque des revendications 1 à 3, dans laquelle la cire est une cire végétale, de préférence choisie parmi la cire de candelilla, la cire de carnauba, la cire du Japon, la cire de sparte, la cire de liège, la cire de guaruma, la cire d'huile de germes de riz, la cire de canne à sucre, la cire d'ouricury, la cire minérale, ou des mélanges de ceux-ci.

6. Pièce moulée rigide pour usage selon l'une quelconque des revendications 1 à 3, dans laquelle la cire est une cire pétrochimique, de préférence de la cire de paraffine, de la cire de stéarine ou des mélanges de ceux-ci.

7. Pièce moulée rigide pour usage selon l'une quelconque des revendications précédentes, dans laquelle les grains de sable enrobés de cire présentent un rapport en poids de sable à cire de 2 : 1 à 25 : 1, de préférence de 4 : 1 à 12 : 1, de manière particulièrement préférée de 6 : 1 à 10 : 1.

8. Pièce moulée rigide pour usage selon l'une quelconque des revendications précédentes, dans laquelle les grains de sable présentent un diamètre de 0,06 à 3 mm, de préférence de 0,1 à 0,5 mm

9. Pièce moulée rigide pour usage selon l'une quelconque des revendications précédentes, dans lequel la démangeaison est causée par une piqûre d'insectes, une allergie, une névrodermite, un dommage et / ou une irritation de la peau, un contact avec une ortie, et / ou un diabète.

10. Pièce moulée rigide pour usage selon l'une quelconque des revendications précédentes, dans laquelle la pièce moulée présente une forme de tige, de préférence avec un diamètre de 1 à 3 cm et / ou une longueur de 3 à 10 cm.

11. Pièce moulée rigide pour usage selon l'une quelconque des revendications précédentes, dans laquelle la pièce moulée rigide présente une forme parallélépipédique ou une forme sphérique, de préférence avec un diamètre de 5 à 10 cm et / ou une longueur de 5 à 10 cm.

12. Pièce moulée rigide pour usage selon l'une quelconque des revendications précédentes, dans laquelle la pièce moulée est rendue rugueuse au moins partiellement avant la mise en contact avec l'endroit qui démange.
